(19) Europäisches Patentamt
European Patent Office
Office européen des brevets

(11) Publication number: 0 124 961 B1

# (12) EUROPEAN PATENT SPECIFICATION

(45) Date of publication of patent specification: 09.12.87

(51) Int. Cl.$^4$: C 07 D 233/90

(21) Application number: 84301159.4

(22) Date of filing: 23.02.84

## (54) A process for the production of 4-carbamoyl-1H-imidazolium-5-olate.

(30) Priority: 11.03.83 US 474410

(43) Date of publication of application:
14.11.84 Bulletin 84/46

(45) Publication of the grant of the patent:
09.12.87 Bulletin 87/50

(84) Designated Contracting States:
AT BE CH DE FR GB IT LI LU NL SE

(58) References cited:
FR-A-2 370 737

JOURNAL OF THE AMERICAN CHEMICAL SOCIETY, vol. 74, no. 11, 11th June 1952, pages 2892-4, Washington, D.C., US; C.S.MILLER et al.: "Substituted imidazoles as precursors of the purines"

(73) Proprietor: WARNER-LAMBERT COMPANY
201 Tabor Road
Morris Plains New Jersey 07950 (US)

(72) Inventor: Cook, Phillip Dan
2333 Georgetown Blvd.
Ann Arbor Michigan 48105 (US)
Inventor: McNamara, Dennis Joseph
1102 Birk Avenue
Ann Arbor Michigan 48103 (US)

(74) Representative: Jones, Michael Raymond et al
HASELTINE LAKE & CO
Hazlitt House 28 Southampton Buildings
Chancery Lane
London WC2A 1AT (GB)

EP 0 124 961 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

The present invention relates to a novel process for the production of 4-carbamoyl-1*H*-imidazolium-5-olate (also referred to as bredinin agylcone and 5-hydroxyimidazole-4-carboxamide) which is a substance having useful pharmacological properties such as antitumor, antirheumatic and antinephritic properties. The product of the process is also useful as a starting material for the production of antibiotic compounds and other compounds.

4-Carbamoyl-1*H*-imidazolium-5-olate was first synthesized by reacting aminomalonamide with triethyl orthoformate at 145°C for two hours; Studies in Imidazoles. II. Imidazo[b] pyrazines, E. Schipper and A. R. Day, *J. Am. Chem. Soc.*, *74*, 350—353 (353), 1952. This method, however, is inefficient because the product obtained from this procedure is contaminated with aminomalonamide, one of the starting materials. Repeated recrystallisation gradually reduce this contaminant, but with a consequent lowering of the yield of pure 4-carbamoyl-1*H*-imidazolium-5-olate product.

A similar method in which aminomalonamide is refluxed with acetic acid in triethyl orthoformate for 0.5 hours is also inefficient, and the product obtained is again contaminated with aminoalonamide: 5-Hydroxy-imidazole-4-carboxamide; T. Atsume, Y. Takebayashi, J. Katsube, and H. Yamamoto; Sumitomo Chemical Co. Ltd., Japan, Kokai; Japanese Pat. No. 76 88,965.

According to the present invention there is provided a process for the production of 4-carbamoyl-1*H*-imidazolium-5-olate (I)

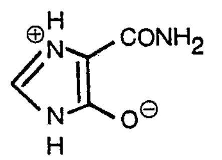

(I)

which process comprises reacting substantially equimolar quantities of aminomalonamide and of a formamidine compound in a liquid medium comprising a low boiling point solvent, wherein the formamidine compound is the formic acid amidine in the form of the free base or as a salt, and wherein the conditions are such that the product can be obtained in a solid form that is directly recoverable from the liquid medium, and is free of aminomalonamide and is recrystallizable.

The compound aminomalonamide has the formula $H_2N—CH(CONH_2)_2$, and formamidine has the formula $CH(=NH)NH_2$.

The use of formamidine acetate in place of triethyl orthoformate or ethyl formimidate and acid catalysts provides a simple, high yield process to pure 4-carbamoyl-1*H*-imidazolium-5-olate. Surprisingly, the condensation is specific for the formic acid amidine; substituted formamidines such as acetamidine and benzamidine hydrochlorides do not yield 2-substituted imidazoles. The process of the present invention also fails to yield 2-heteroatom substituted imidazoles by the use of substituted amidines such as guanidine or methylthiopseudourea acid salts.

The condensation is specific for the formic acid amidine, either as the free base or as the salt such as the hydrochloride salt or acetic acid salt, referred to hereinafter as the formamidine compound. An acid salt of formamidine and an equivalent amount of base such as triethylamine, sodium methoxide, or potassium carbonate also provides 4-carbamoyl-1*H*-imidazolium-5-olate, according to the invention. Preferably, the process uses low boiling point alcohol solvent (e.g. ethanol or methanol) rather than a high boiling point solvent such as triethyl orthoformate, the latter of which also served in the prior art as a reactant. Unlike other processes, where aminomalonamide starting material is the principal contaminant and is difficult to remove, the present reaction goes to completion. Further, in the present process the desired product precipitates out directly. The precipitated material is relatively pure and, by only one recrystallization, the product is rendered analytically pure, whereas repeated recrystallization or ion exchange chromatography is required in the processes described in the prior art. Finally, the process provides a high yield of pure 4-carbamoyl-1*H*-imidazolium-5-olate.

The reaction conditions are subject to some variation. For good results, approximately equimolar ratios of aminomalonamide and of the formamidine compound are employed; a substantial excess of aminomalonamide is avoided.

The reaction is carried out in a compatible solvent, preferably a relatively low boiling point solvent comprising alcohol, preferably methanol or ethanol, and more preferably ethanol alone. For the best results, formamidine acetate is used, in an amount such that there are from 2 to 4 litres of ethanol for each mole of formamidine acetate. The reaction is conveniently carried out by maintaining the reactants as a suspension in the solvent and at the reflux temperature of the reaction medium. The reaction is substantially complete within short periods, typically within about one hour, and thereafter the resulting suspension of product can be stirred at room temperature overnight to ensure completion of the reaction. The product is obtained as an off-white suspension that can conveniently be recovered as a solid by filtration. Recrystallization is achieved using water as a solvent.

The invention may best be illustrated by reference to the following example.

Example

*4-Carbamoyl-1H-imidazole-5-olate.* A suspension of 105.5 g of aminomalonamide and 112.5 g of formamidine acetate in 4.0 litres of ethanol was heated under reflux for one hour. The resulting off-white suspension was stirred at room temperature overnight and filtered. Recrystallization of the solid from approximately 2 litres of water, followed by drying under reduced pressure at 100°C, gave 93 g (78 percent) of the product as a dark blue-grey solid, mp 255°C. PMR spectroscopy of the product in deuterated dimethylsulphoxide indicated an absence of aminomalonamide. Thin-layer-chromatography ($SiO_2$,$CH_3CN$:0.2*M* $NH_4Cl$, volume ratio of 3:1) revealed an homogeneous product with an $R_f$ of 0.34.

Analysis calculated for $C_4H_5N_3O_2 \cdot 0.35\ H_2O$: C, 36.01; H, 4.31; N, 31.50; $H_2O$, 4.72;
Found: C, 36.06; H, 4.13; N, 31.53; $H_2O$, 5.08.

**Claims**

1. A process for the production of 4-carbamoyl-1*H*-imidazolium-5-olate, which process comprises reacting substantially equimolar quantities of aminomalonamide and of a formamidine compound in a liquid medium comprising a low boiling point solvent, wherein the formamidine compound is the formic acid amidine in the form of the free base or as a salt, and wherein the conditions are such that the product can be obtained in a solid form that is directly recoverable from the liquid medium, is free of aminomalonamide and is recrystallizable.

2. A process according to Claim 1, wherein the reaction is carried out at the reflux temperature of the reaction medium.

3. A process according to Claim 1 or 2, wherein the liquid medium comprises one or more alcohol.

4. A process according to Claim 3, wherein the liquid medium is ethanol.

5. A process according to any preceding claim, wherein the formamidine compound is formamidine acetate.

6. A process according to Claim 5 when appendant to Claim 4, wherein the quantity of ethanol employed is in the range from 2 to 4 litres for each mole of formamidine acetate.

7. A process according to any preceding claim, wherein the product is recovered in solid form from the reaction medium and, subsequently, recrystallized from water.

**Patentansprüche**

1. Verfahren zur Herstellung von 4-Carbamoyl-1H-imidazolium-5-olat, welches Verfahren die Umsetzung im wesentlichen äquimolarer Mengen von Aminomalonamid und einer Formamidinverbindung in einem flüssigen Medium, enthaltend ein Lösungsmittel mit niedrigem Kochpunkt, umfaßt, worin die Formamidinverbindung das Ameisensäureamidin in Form der freien Base oder als Salz ist, und worin die Bedingungen derart sind, daß das Produkt in fester Form, die direkt aus dem flüssigen Medium gewonnen werden kann, erhältlich, frei von Aminomalonamid und umkristallisierbar ist.

2. Verfahren nach Anspruch 1, worin die Reaktion bei der Rückflußtemperatur des Reaktionsmediums durchgeführt wird.

3. Verfahren nach Anspruch 1 oder 2, worin das flüssige Medium einen oder mehrere Alkohole umfaßt.

4. Verfahren nach Anspruch 3, worin das flüssige Medium Äthanol ist.

5. Verfahren nach einem der vorhergehenden Ansprüche, worin die Formamidinverbindung Formamidinacetat ist.

6. Verfahren nach Anspruch 5 in Abhängigkeit von Anspruch 4, worin die verwendete Äthanolmenge im Bereich von 2 bis 4 Liter pro Mol Formamidinacetat liegt.

7. Verfahren nach einem der vorhergehenden Ansprüche, worin das Produkt in fester Form aus dem Reaktionsmedium gewonnen und anschließend aus Wasser umkristallisiert wird.

**Revendications**

1. Un procédé de production de 4-carbamoyl-1H-imidazolium-5-olate, consistant à faire réagir des quantités pratiquement équimolaires d'aminomalonamide et d'un composé à base de formamidine dans un milieu liquide comprenant un solvant de bas poids d'ébullition, dans lequel le composé à base de formamidine consiste en l'amidine de l'acide formique sous forme de base libre ou d'un sel, les conditions étant telles que le produit est obtenu sous forme solide directement récupérable du milieu liquide, est exempt d'aminomalonamide et est recristallisable.

2. Un procédé selon la revendication 1, dans lequel la réaction est mise en oeuvre à la température du reflux du milieu réactionnel.

3. Un procédé selon la revendication 1 ou la revendication 2, dans lequel le milieu liquide comprend un ou plusieurs alcools.

4. Un procédé selon la revendication 3, dans lequel le milieu liquide est formé d'éthanol.

5. Un procédé selon une quelconque des revendications précédentes dans lequel le composé à base de formamidine est l'acétate de formamidine.

6. Un procédé selon la revendication 5 en relation avec la revendication 4, dans lequel la quantité

d'éthanol employée est comprise entre 2 et 4 litres pour chaque mole d'acétate de formamidine.

7. Un procédé selon l'une quelconque des revendications précédentes, dans lequel le produit est récupéré du milieu liquide sous forme solide et est ultérieurement recristallisé dans de l'eau.